# EUROPÄISCHE PATENTSCHRIFT

(11) **EP 0 946 816 B1**
(45) Veröffentlichungstag und Bekanntmachung des Hinweises auf die Patenterteilung: **11.09.2002**
(21) Anmeldenummer: 97952750.4
(22) Anmeldetag: 19.12.1997
(51) Int. Cl.: D21C 7/12, G01N 33/34

(54) **VERFAHREN UND VORRICHTUNG ZUR PROZESSFÜHRUNG BEI DER HERSTELLUNG VON ZELLSTOFF**
METHOD AND DEVICE FOR CONDUCTING A PROCESS IN THE PRODUCTION OF CELLULOSE
PROCEDE ET DISPOSITIF POUR CONDUIRE UN PROCESSUS LORS DE LA FABRICATION DE CELLULOSE

(30) Priorität: 20.12.1996 DE 19653530
(43) Veröffentlichungstag der Anmeldung: 06.10.1999
(73) Patentinhaber: SIEMENS AKTIENGESELLSCHAFT, 80333 München (DE)
(72) Erfinder: LAMPE, Uwe, D-21614 Buxtehude (DE); FURUMOTO, Herbert, D-91052 Erlangen (DE); ZEINER, Gerhard, D-70563 Stuttgart (DE); ROTH, Christoph, D-81739 München (DE)
(86) Internationale Anmeldenummer: DE9702990
(87) Internationale Veröffentlichungsnummer: WO98028487

(56) Entgegenhaltungen:
- WO-A-94/01769
- WO-A-96/12183
- DE-A- 19 510 008
- US-A- 4 978 425
- US-A- 5 486 915

## Beschreibung

Die Erfindung bezieht sich auf ein Verfahren zur Prozeßführung und zur Prozeßoptimierung bei der Herstellung von Zellstoff durch Kochen von Ausgangsstoffen mit Kochflüssigkeit in einem Zellstoffkocher unter Einsatz wenigstens eines Zustandsmodells und/oder Prozeßmodells. Daneben bezieht sich die Erfindung auf eine Vorrichtung zur Durchführung des Verfahrens.

Die Herstellung von Zellstoff durch den sogenannten Zellstoffaufschluß erfolgt durch Kochung unter Einsatz von entsprechenden Kochchemikalien entweder in einem kontinuierlichen oder in einem diskontinuierlichen Prozeß. An die Prozeßführung, insbesondere die Prozeßführung des Kochers, werden dabei erhebliche Anforderungen gestellt.

Die Prozeßführung bei der Herstellung von Zellstoff ist schwierig, da wichtige Qualitätsparameter des Zellstoffes, beispielsweise die Kappazahl, die Viskosität oder die Reißlänge erst am fertigen Produkt ermittelt werden können. Da diese Werte im Labor mit Zeitverzögerung gemessen werden, kann eine zwischenzeitliche Fehlproduktion nicht ausgeschlossen werden. Um die Gefahr einer Fehlproduktion zu vermeiden, wird daher üblicherweise der Produktionsprozeß mit einem größeren Sicherheitsspielraum hinsichtlich der Produktqualität gefahren, als es eigentlich notwendig wäre.

Beim Stand der Technik werden häufig folgende Strategien angewandt:
- Der Prozeß wird nach Erfahrungswerten gesteuert. Regeleingriffe sind nur in geringem Umfang möglich. Die Produktqualität kann nur im nachhinein bestimmt werden. Eine große Streuung der Qualitätsparameter ist daher häufig nicht vermeidbar, insbesondere wenn die Qualität der eingesetzten Rohstoffe, z.B. das Holz, oder die Qualität der eingesetzten Chemikalien, z.B. die Weißlauge stark schwankt.
- Es erfolgt ein analytisches Messen einzelner Komponenten der Kochflüssigkeit mit beispielsweise Titrationsautomaten.
- Es wird auch bereits versucht, den Prozeß über online meßbare Hilfsgrößen zu regeln. Da die Zahl der mit nicht zu hohen Aufwand meßbaren Hilfsgrößen begrenzt ist und zudem häufig nur ein empirischer Zusammenhang mit den Zielgrößen besteht, sind weitere Optimierungen des Kochprozesse nur sehr schwer möglich.

Daneben wurde bereits vorgeschlagen, Infrarotspektroskopie zur Prozeßbeurteilung vorzusehen, wozu eine Reihe von Veröffentlichungen existiert. Aus der DE-A-195 10 009 ist ein Verfahren zur Prozeßführung einer Papiermaschine bekannt. Dabei werden ausgewählte spektrale Kennwerte für die Messung von Produktqualitätsparametern verwendet. Speziell zur Prozeßführung beim Stoffauflauf einer Papiermaschine werden neuronale Netze eingesetzt, die an den im Labor gemessenen Produktparametern trainiert wurden. Weiterhin ist aus der DE-A-195 10 008 ein Verfahren zur Prozeßführung bei der Zellstoff- und Papierherstellung bekannt, bei dem spektrale Kennwerte unterschiedlicher Wellenlängen zur Bestimmung der Ausgangsstoffe und zur Bewertung der Rohstoffqualität abgeleitet werden. Ebenfalls insbesondere mit neuronalen Netzen sollen dort Korrektursignale für die anlagentechnischen Regel- oder Steuereinrichtungen ermittelt werden. Bei diesem Verfahren erfolgt die Meßsignalaufnahme direkt am Rohstoff "Holz", wie Holzhackschnitzel oder Holzfasern, oder direkt am Rohstoff "Altpapier".

Gemäß der US-A-5 378 320 wird bei der Sulfat-Zellstoff-Herstellung über IR-Spektroskopie bei ausgewählten Wellenzahlen die Absorption gemessen und über Korrelationen verschiedene Alkalikonzentrationen ermittelt. Weiterhin wird vorgeschlagen, mit spektroskopischen Messungen den Ligningehalt von Zellstoff zu bestimmen.

Schließlich ist aus der DE-A-36 16 051 ein Verfahren zur Kontrolle des Aufschlusses von Lignozellulosen mit Hilfe von FTIR - Spektroskopie bekannt. Durch Messung im MIR-(Mittleren Infrarot)-Bereich wird der Restligninanteil und die Konzentration der Kochchemikalien bestimmt, z.B. der Gehalt an freiem SO₂ bei der Sulfitkochung.

Davon ausgehend ist es Aufgabe der Erfindung, die Prozeßführung bei der Zellstoffherstellung weiter zu verbessern und eine zugehörige Vorrichtung zu schaffen.

Die Aufgabe ist bei einem Verfahren der eingangs genannten Art erfindungsgemäß mit den Merkmalen des Patentanspruches 1 gelöst, wobei vorteilhafte Weiterbildungen in den abhängigen Ansprüchen angegeben sind. Die zugehörige Vorrichtung ist im einzigen Sachanspruch angegeben.

Bei der Erfindung werden vorteilhafterweise an der Kochflüssigkeit spektroskopische Messungen durch Absorption, Emission, Lumineszenz im Spektralbereich von 0,1 µm bis 400 µm, vorzugsweise zwischen 0,4 µm und 100 µm, durchgeführt. Die kontinuierlich Spektren werden mit geeigneten Rechenverfahren ausgewertet und ermöglichen eine Aussage über die zu erwartende Zellstoffqualität und die chemische Zusammensetzung der Kochlauge. Auf der Basis dieser Aussagen kann in den Prozeß regelnd eingegriffen werden, z. B. durch Änderung der Kochungsdauer bzw. der Verweilzeit in der Kochzone und der Kochtemperatur, Zudosierung von Kochchemikalien usw. Das Verfahren mit Erfassung der kontinuierlichen Spektren ist alternativ für den Sulfit- oder Sulfatprozeß einsetzbar.

Vorteilhaft ist insbesondere für die Zellstoffherstellung die Vergleichsmäßigung der Produktqualität, Erhöhung der Ausbeute und der Produktionsmenge, die Reduzierung des Chemikalienund/oder Energieeinsatzes und auch die Einsparung von Hilfsstoffen in der an die Zellstoffherstellung nachfolgenden Bleichstufe.

Die Erfindung ist gegenüber dem angegebenen Stand der Technik in wesentlichen Punkten, wie dem Meßort, der Meßmethode, der Verarbeitung der Spektren und der Modellbildung, weiterentwickelt. Die Erfindung ist im Rahmen der Zellstoffkochung sowohl beim kontinuierlichen als auch beim diskontinuierlichen Kocher anwendbar.

Sofern die Messung in der Kochflüssigkeit erfolgt, ist beim diskontinuierlichen Verfahren die Umpumpleitung eine geeignete Meßstelle. Bei den kontinuierlichen Verfahren sind dagegen insbesondere die einzelnen Zirkulationen der Kochflüssigkeit (C5-, C6-) oder die Chemikalienzufuhr und der Ablauf der Schwarzlauge (C15-, C16-Leitungen) geeignet.

Sofern mit elektromagnetischen Wellen im Bereich der Wellenlängen zwischen 100 nm und 400 µm, vorzugsweise im Bereich von 0,4 µm bis 100 µm, gearbeitet wird, können Absorptions-, Emissions-, Lumineszenz- oder Ramanspektren gemessen werden. Die Absorptionsspektroskopie kann in Transmission, diffuser Reflexion oder gedämpfter Totalreflexion (ATR = atennuated total reflection) erfolgen. Die Anregung zur Lumineszenz kann z.B. durch die Einstrahlung von elektromagnetischer Strahlung erfolgen (z.B. UV-Strahlung) oder durch eine spezifische chemische Reaktion (Chemolumineszenz), die Anregung der Emission dagegen z.B. durch Bestrahlung mit Elektronen erfolgen. Bei Messung im Bereich des Infraroten (800 nm bis 20 µm) kann vorzugsweise die Fourier-Transformations-Infrarot-Spektroskopie (FTIR) eingesetzt werden. Bei inhomogenen Proben erfolgt zum Plausibilitätsvergleich die spektroskopische Messung mehrfach.

Vorzugsweise werden die Spektren wie folgt vorverarbeitet:
- durch Fourier-Transformation.
- Bei der Messung der Absorption durch diffuse Reflexion durch Umrechnung in Kubelka-Munk-Einheiten und Korrektur von Mehrfachstreueffekten
- durch Normierung und Glättung der Spektren
- durch Ermittlung von für die Modellbildung ungeeigneten Spektren. Die Ausschaltung ungeeigneter Messungen kann z.B. durch Vergleich mit Referenzspektren erfolgen
- durch Bildung von Mittelwerten bei mehreren Spektren zu einer Probennahme
   Nach diesen ersten Verarbeitungsschritten werden aus Spektren ganz oder abschnittsweise folgende rechnergestütze Verfahren zur Ermittlung von Kenngrößen angewandt, wobei die Beschreibung der Spektren im wesentlichen durch ihre Hauptkomponenten erfolgt,
- Hauptkomponentenanalyse (PCA)
- "Partial Least Square" (PLS)-Verfahren
- Neuronale Netze
- Analytische Beschreibung der Spektren, z.B. im Bereich des IR durch Lage, Intensität und Breite der wichtigsten Absorptions- oder Emissionspeaks, Ermittlung dieser Größen z.B. mit einfachen Minimum-/Maximum-Verfahren oder der 2. Ableitung.

Die Kenngrößen werden zur Modellierung der gewünschten Qualitätsparameter herangezogen.

Die Zustandsmodelle zur Berechnung der Qualitätsparameter können bei ausreichend großer Zahl von Daten auf der Basis von neuronalen Netzen, Fuzzy-Systemen, Multilinearen Regressionsmodellen bzw. Kombinationen daraus strukturiert sein. Alternativ zu rein datengetriebenen Modellen sind auch kombinierte Modelle möglich, bei denen zusätzlich analytisches Wissen eingebracht wird. Auf die gleiche Weise und mit den gleichen Mitteln können auch die Prozeßmodelle aufgebaut werden.

Die Aufstellung der Modelle erfolgt mit Labormessungen am Zwischen- und Endprodukt. Das Training der Modelle erfolgt jeweils auf der Grundlage der Laborwerte und kann in bestimmten Zeitabständen wiederholt werden, wobei auch ein nur partielles Nachlernen möglich ist.

Eine in der Spektrenvorverarbeitung integrierte Prüfung auf Modellgültigkeit ("Novelty Detection") kann im laufenden Prozeß rechtzeitig die Notwendigkeit einer neuen Trainingsphase anzeigen.

Weitere Einzelheiten und Vorteile der Erfindung ergeben sich aus der nachfolgenden Figurenbeschreibung von Ausführungsbeispielen anhand der Zeichnung. Es zeigen
- Figur 1: ein Schema der Meßstellen bei der Kochung von Zellstoff im kontinuierlichen Kocher,
- Figur 2: ein entsprechendes Schema für einen diskontinuierlichen Kocher,
- Figur 3a und 3b: kontinuierliche Spektren von optischen Messungen an Kochflüssigkeit und von mechanischen Messungen an Fasern,
- Figur 4: ein Zustandsmodell für die Qualitätsparameter bei einem Kocher,
- Figur 5: ein Prozeßmodell für die kontinuierliche Zellstoffkochung,
- Figur 6: ein Prozeßmodell für die diskontinuierliche Zellstoffkochung,
- Figur 7: ein dynamisches Prozeßmodell zur Ermittlung der sogenannten Kappazahl,
- Figur 8: den schematischen Aufbau einer Prozeßoptimierung zum Steuern der Zellstoffkochung,
- Figur 9: eine Variante zu Figur 8 unter Einbeziehung des dynamischen Modells gemäß Figur 7,
- Figur 10: die Vorverarbeitung und Verdichtung der Spektren und
- Figur 11: eine Vorrichtung zur optimierten Prozeßführung bei der Zellstoffherstellung.

Die Figuren werden nachfolgend teilweise gemeinsam beschrieben. Gleiche bzw. gleichwirkende Teile haben sich entsprechende Bezugszeichen.

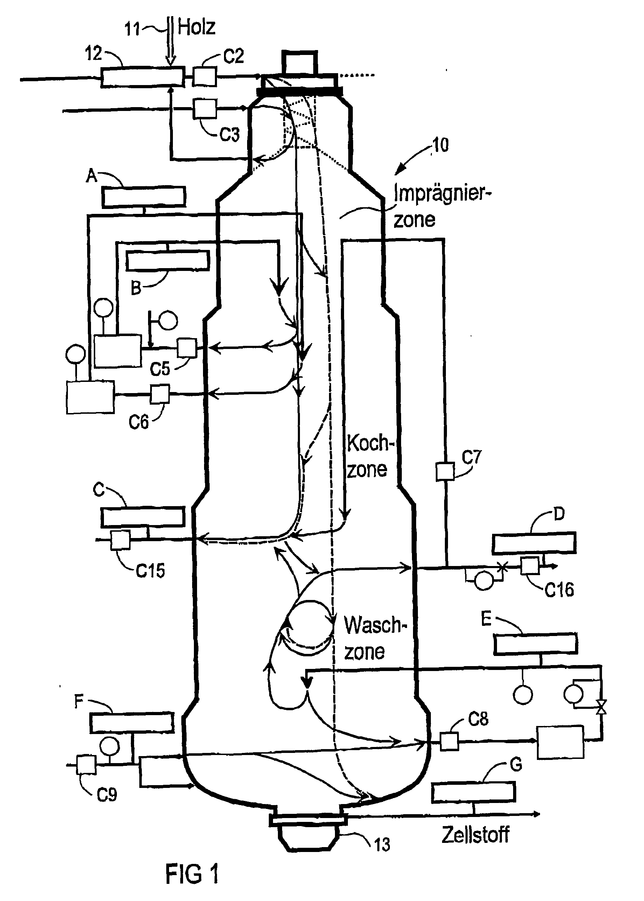

In Figur 1 ist der Aufbau eines kontinuierlich arbeitenden Kochers 10 dargestellt, wobei in vorliegendem Zusammenhang das Meßstellenschema verdeutlicht wird. Mit 11 ist der Einlaß für das Holz, mit 12 die Zuleitung für die Kochflüssigkeit und mit 13 der Auslaß für den fertigen Zellstoff bezeichnet. Aufgrund des kontinuierlichen Betriebes ist ein solcher Kocher vergleichsweise komplex aufgebaut. Im Kocher 10 sind bestimmte Bereiche herausgegriffen, die für den Betrieb des Kochers 10 wesentlich sind. Dies sind einerseits die Imprägnierzone, die Kochzone und die Waschzone. Dazu werden einzelne Zirkulationen an entsprechenden Punkten gemessen. Diese Punkte sind in Figur 1 mit C2, C3, C5 bis C9 sowie C15 und C16 bezeichnet. Dabei kennzeichnen C2 den Eintrag des Holzes und der Kochflüssigkeit, C3 die Imprägnierzone, C5 bis C7 und C15 die Kochzone sowie C8, C9 und C16 die Waschzone. An entsprechenden Stellen sind Spektrometer zur Aufnahme von Spektren A, B, C, D, E, F und G angebracht.

Bei der Zellstoffherstellung speziell nach dem kontinuierlichen Sulfatprozeß werden in den Zirkulationen der Kochzone, wie beispielsweise der C5- und C6-Zirkulation des kontinuierlichen Kochers, online in der Schwarzlauge vorzugsweise im Bereich des IR (Infrarot: 1 - 25 µm) spektroskopische Messungen durchgeführt. Die Messung kann z.B. mit Hilfe einer ATR-Sonde und einem FTIR-Spektrometer durchgeführt werden.

Die gemessenen Spektren werden als Vorverarbeitung geglättet und normiert. Anschließend kann eine Zerlegung in die Hauptkomponenten und/oder die Identifikation wichtiger Peaks erfolgen. Aus den Hauptkomponenten können anschließend mit Modellen, z.B. nach Methoden der multilinearen Regression, die Konzentrationen folgender Größen berechnet werden: Gelöstes Lignin, Effektiv Alkali, Sulfidität, Carbonat, Sulfat, Thiosulfat, Carbonsäuren und gelöste Kohlehydrate (sog. Hemizellulosen).

Letztere werte werden neben anderen Werten bekanntermaßen in Prozeßmodellen für die Berechnung der Kappazahl, der Ausbeute, der Viskosität oder der Reißlänge als Qualitätsparameter des erzeugten Zellstoffes verwendet.

Ein regelnder Eingriff ist an verschiedenen Punkten des Kochprozesses denkbar, und zwar durch Nachdosieren von Chemikalien, durch Temperaturvariationen, durch Änderungen in den Zirkulationen.

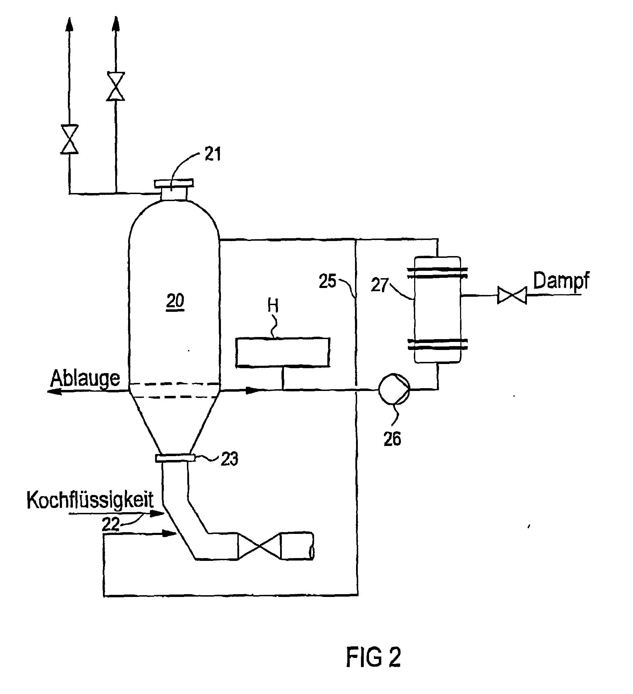

In Figur 2 ist ein Zellstoffkocher 20 wiedergegeben, der diskontinuierlich nach dem batch-Verfahren arbeitet. Aufgrund des Temperatur- Zeit- Verlaufes mit weitgehend vorgegeben Temperaturprofil ist hier die Prozeßführung einfacher als beim kontinuierlichen Kocher gemäß Figur 1. Wesentlich für den Aufbau und Betrieb des diskontinuierlichen Zellstoffkochers 20 sind hier der Füllstutzen 21 für das Holz einerseits sowie die Zuleitung 22 für die Kochflüssigkeit andererseits und der Ausgangsstutzen 23 für das periodische Entleeren des Zellstoffes. Neben diversen Ein- und Ausgangsleitungen ist wesentlich ein Kreislauf 25 mit einer Umwälzpumpe 26, mit der eine Zirkulation der Kochflüssigkeit über einen Wärmetauscher 27 realisiert ist. In der Leitung vor der Umwälzpumpe ist ein Spektrometer angebracht, mit welchem das Spektrum H aufgenommen wird.

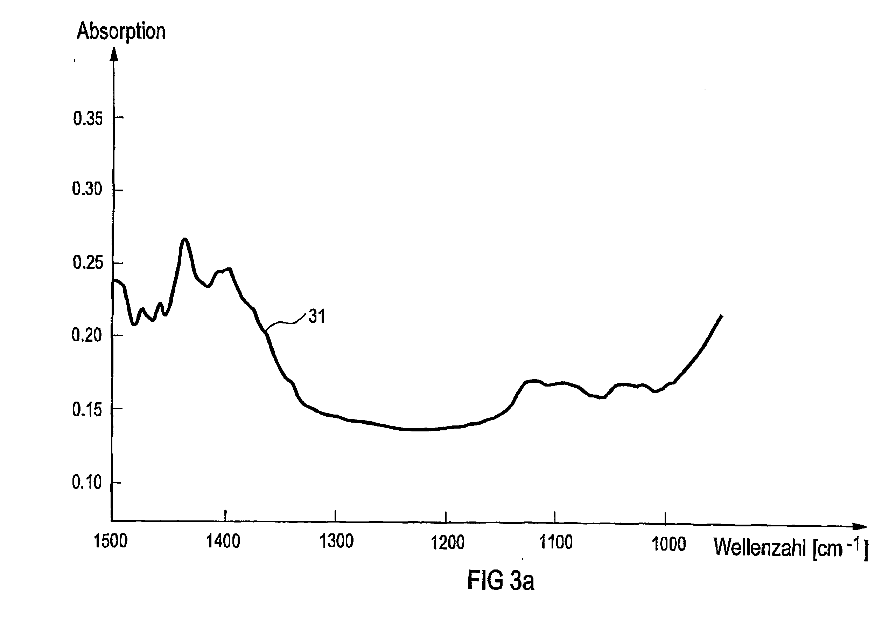

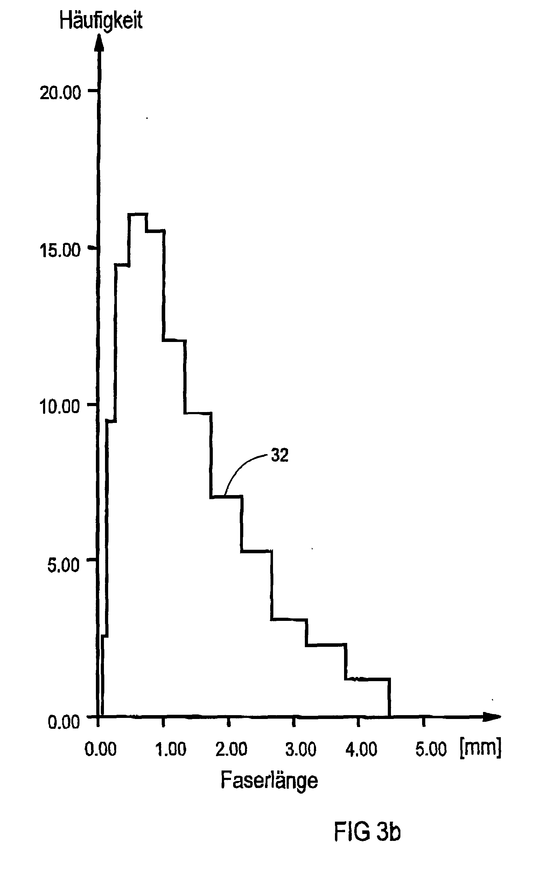

In Figur 3a kennzeichnet 31 beispielhaft den Verlauf eines IR-Spektrums von Kochflüssigkeit im Wellenzahlbereich 1500 bis 900 cm⁻¹. In Figur 3b ist dagegen das Spektrum einer Faserlängenverteilung des hergestellten Zellstoffs dargestellt, wie es sich bei einer beispielhaften Messung der mechanischen Eigenschaften ergibt. Die Grenzlinie 32 der Balkenverteilung gemäß Figur 3b gibt im wesentlichen einen Verlauf entsprechend einer sogenannten Bernoulli-Verteilung wieder. Die Verteilung der Siebfraktionen einzelner Fasern von Zellstoff hat einen ähnlichen Verlauf.

Die anhand der Figuren 3a bzw. 3b erhaltenen Spektren werden mittels unterschiedlichster mathematischer Methoden aufbereitet. Dabei geht es im wesentlichen um eine Vorverarbeitung der Spektren, um das Einbringen von analytischem Wissen, um eine mögliche Ausreißererkennung und eine bestimmungsgemäße Ableitung von Kenngrößen.

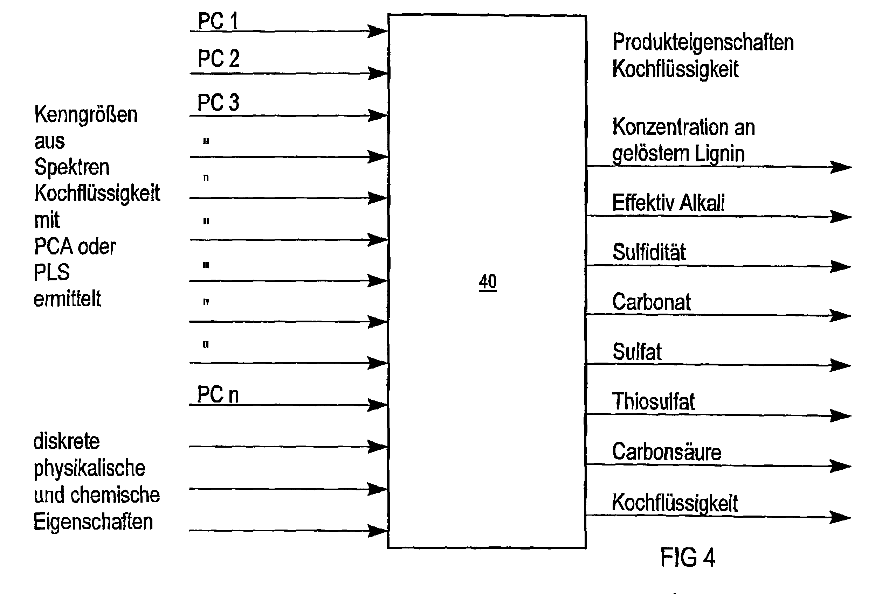

In Figur 4 bedeuten 40 ein Zustandsmodell der Kochflüssigkeit, aus dem ihre Eigenschaften, wie z.B. gelöstes Lignin, Effektiv-Alkali, Sulfidität, Carbonat-, Sulfat-, Thiosulfat-, Carbonsäuren- sowie auch gelöste Kohlehydrate (Hemizellulose)-Konzentration berechnet werden. Daraus können die wesentlichen Qualitätsparameter für den Zellstoff wie Kappazahl und Viskosität abgeleitet werden. Dazu werden aus den Spektren entweder mit der sogenannten Hauptkomponentenanalyse (PCA = principal component analysis) oder mit der sogenannten PLS-Methode (partial least square) Kenngrößen ermittelt und in das Modell eingegeben. Weiterhin werden auch diskrete mechanische oder chemische Eigenschaften ausgewählt und in das Zustandsmodell 40 eingegeben. Das Zustandsmodell kann auch allein mit aus den kontinuierlichen Spektren ermittelten Kenngrößen gebildet werden.

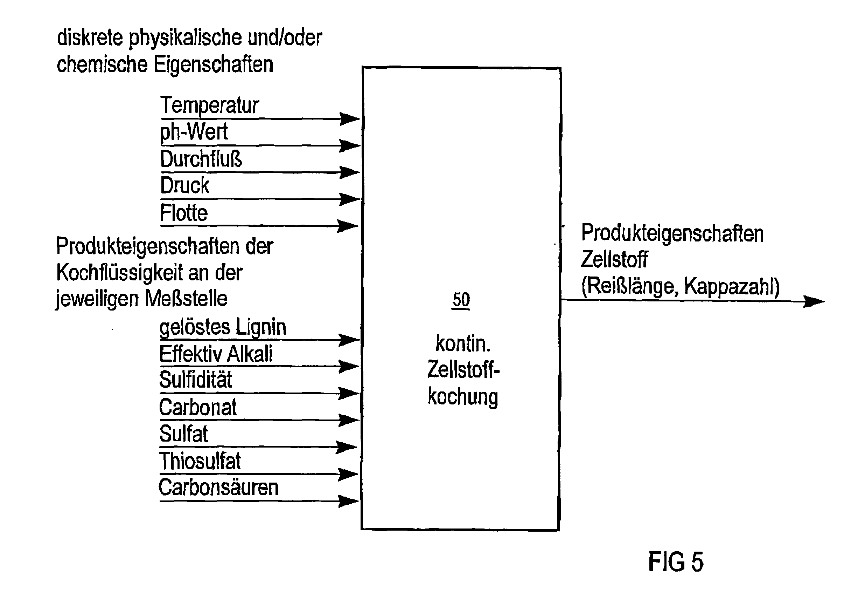

In Figur 5 ist ein Prozeßmodell einer kontinuierlichen Kochung mit 50 bezeichnet. Eingegeben werden beispielhaft diskrete physikalische und chemische Eigenschaften, wie z.B. Temperatur, pH-Wert, Druck, Flottenverhältnis, Chemikalienkonzentrationen, und die Zustandsgrößen der Kochflüssigkeit als Ausgabe der Zustandsmodelle bspw. gemäß Figur 4. Auch das Prozeßmodell kann ggfs. allein mit den aus den kontinuierlichen Spektren ermittelten Kenngrößen gebildet werden.

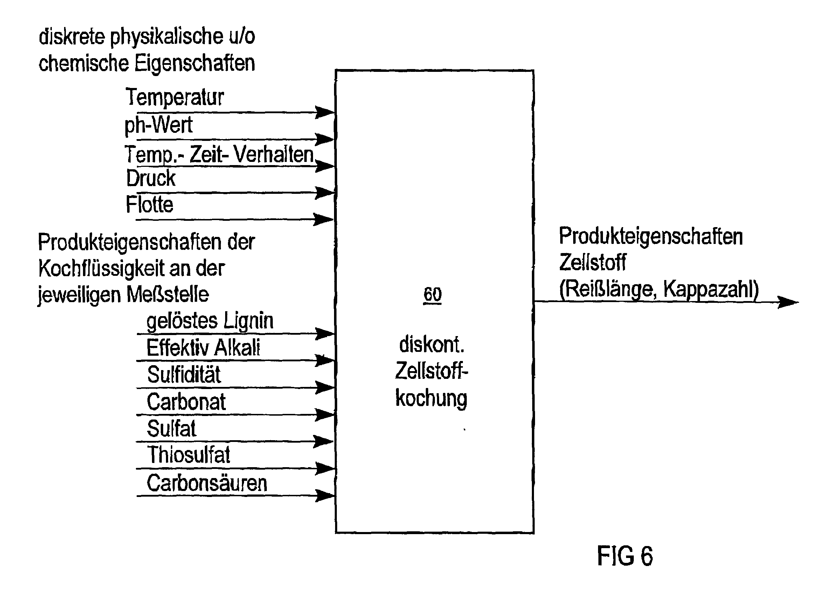

In Figur 6 ist ein entsprechendes Prozeßmodell 60 speziell für einen diskontinuierlichen Kocher gemäß Figur 2 angegeben. Sofern diskrete physikalische und/oder chemische Eigenschaften verwendet werden sollen, können neben dem optischen Spektrum beispielsweise das Flottenverhältnis, der Aktivalkalianteil, die Sulfidität und/oder die Kochtemperatur am Meßort angegeben werden, was in Figur 6 dargestellt ist. Auch die Holzsorte bzw. der Holzsortenanteil und der pH-Wert beim Sulfitaufschluß kann eingegeben werden. Im Unterschied zum kontinuierlichen Kochprozeß mit einem ständigen Durchfluß der Stoffe ist beim baten-Prozeß das Temperatur-Zeit-Verhalten während eines singulären Kochzyklus' von entscheidender Bedeutung als Eingangsgröße. Auch hier ergibt sich daraus wiederum die Reißlänge bzw. die Kappazahl des Zellstoffes.

Für jeden zu berechnenden Qualitätsparameter sind zweckmäßigerweise eigene Modelle zu erstellen. Zur Erhöhung der Vorhersagegenauigkeit werden für jeden Parameter Teilmodelle gebildet. Die Zuordnung zu den Teilmodellen erfolgt auf der Basis der in der Kochung eingesetzten Holzsorten und der vorgegebenen Sollwerte der Qualitätsparameter. Bei einem Einsatz von Holzmischungen können entsprechende Kombinationsmodelle berechnet werden, z.B. lineare Mischung der Modelle entsprechend dem Anteil in der Holzmischung. Die vorhergesagten Werte sind als Ergebnis eines Kombinationsmodelles zu kennzeichnen.

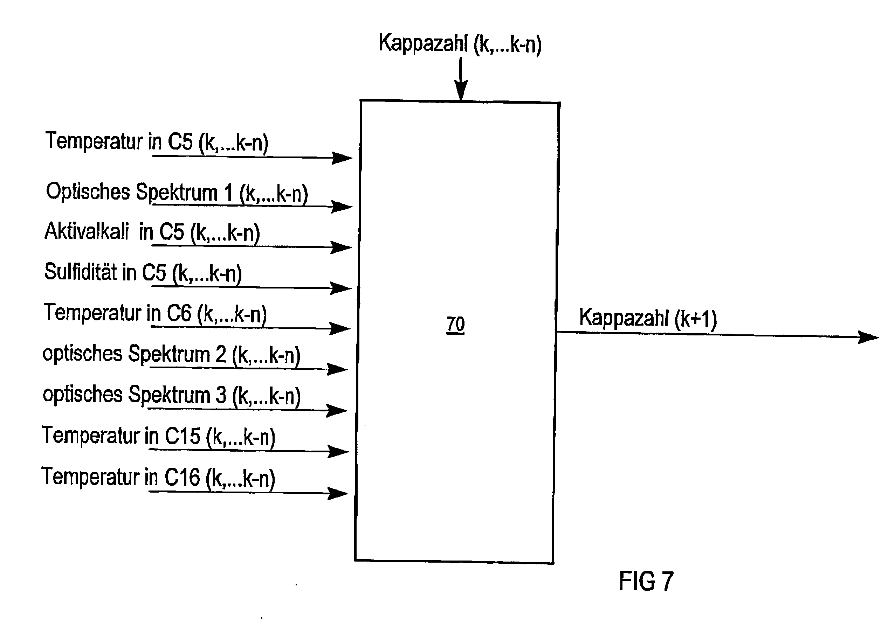

Gemäß Figur 7 kann das Prozeßmodell gemäß Figur 5 auch als dynamisches Prozeßmodell 70 konzipiert sein. Die Eingangsgrößen sind hier entsprechend den Eingangsgrößen in Figur 5 jeweils zu diskreten Zeitpunkten k, ..., (k-n), angegeben. Entsprechendes gilt für die Kappazahl. Daraus bestimmt das dynamische Prozeßmodell 70 insbesondere die Kappazahl zum Zeitpunkt (k+1).

Bei der Aufstellung der Modelle kann von neuronalen Netzen und/oder von Fuzzy-Verfahren Gebrauch gemacht werden. Insbesondere geht es darum, die Gültigkeit der Modelle zu validieren, was durch ein online-Training der einzelnen Modelle bzw. der Teilmodelle erfolgen kann. Dabei kann es für die Praxis wichtig sein, durch rechnergestützte Auswahl aller informationstragenden Daten eine Überprüfung der jeweils erhaltenen Ergebnisse vorzunehmen. Dieses Verfahren wurde als sog. "Novelty Detection" vorgeschlagen und ermöglicht, neue Datensätze in das Auswerteverfahren einzubringen. Bei Vorliegen nicht konsistenter Ergebnisse ist ein Nachtrainieren der Modelle notwendig.

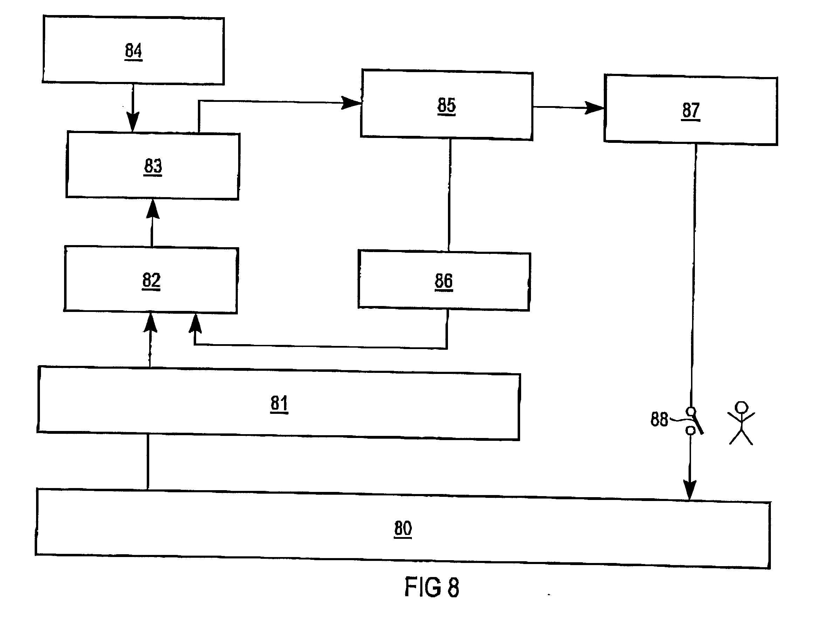

Die vorhandenen Größen werden zur Prozeßführung eingesetzt. Dafür ist in Figur 8 der Prozeß allgemein mit 80 bezeichnet, woraus sich der aktuelle Prozeßzustand anhand der Spektren mit 81 ergibt. Das prozeßmodell ist hier mit 82 bezeichnet, aus dem die Daten in eine Einheit zur Kostenfunktion 83 gegeben werden, die gleichzeitig mit Daten für Kosten und Preise aus der Einheit 84 beaufschlagt wird. Ein Optimierer 85 ermittelt daraus die Stellgrößen 86, die in das Prozeßmodell 82 zurückgekoppelt werden und weiterhin die optimalen Stellgrößen 87 zur Prozeßführung. Diese können über einen Schalter 88 vom Anlagenfahrer durchgeschaltet werden.

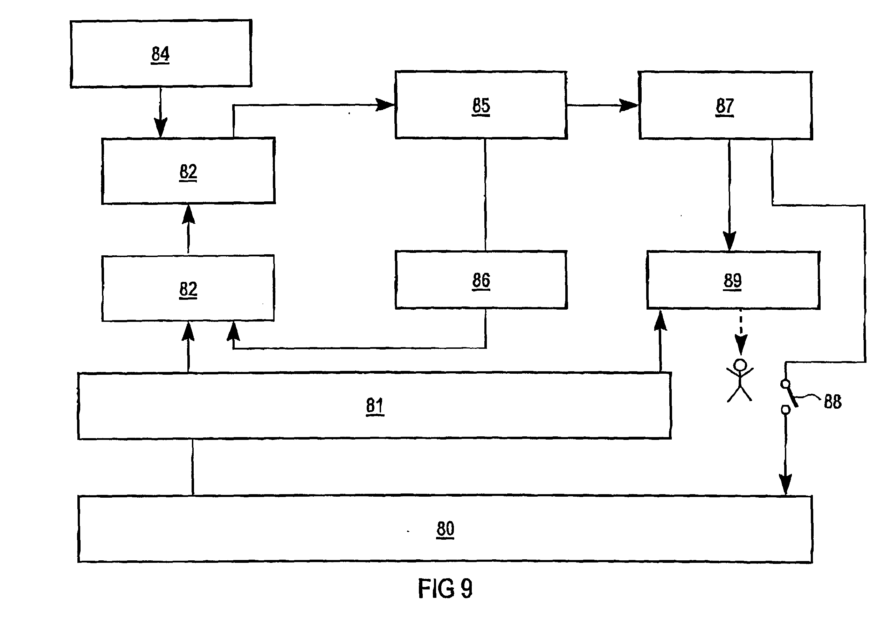

Entsprechendes ergibt sich aus Figur 9 für eine Prozeßführung, bei der gleichermaßen ein dynamisches Modell entsprechend der Figur 7 verwendet wird. Hier ist zusätzlich eine Einheit 89 mit dem dynamischen Modell vorhanden, in die der aktuelle Prozeßzustand einerseits und die optimalen Stellgrößen andererseits angegeben werden.

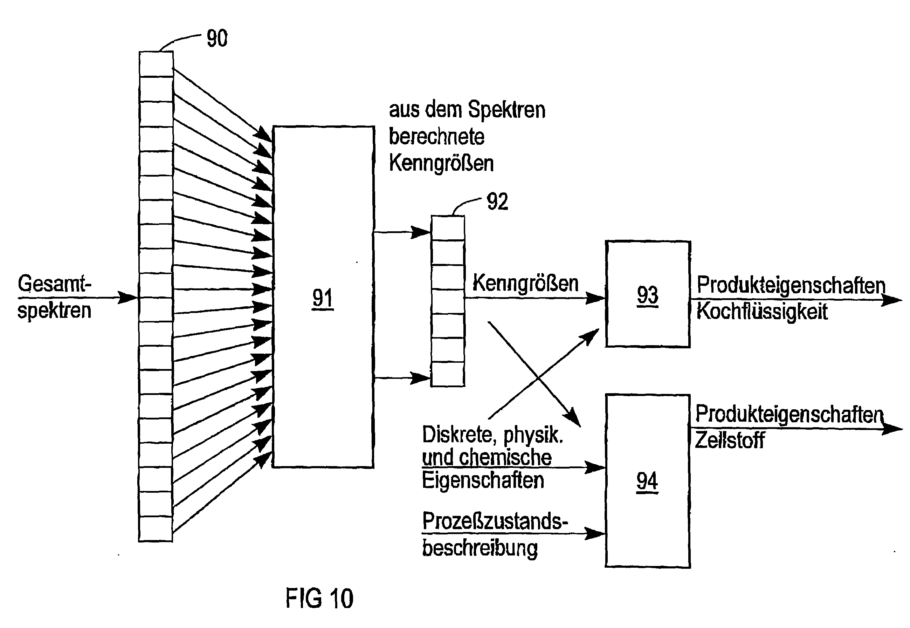

In Figur 10 ist dargestellt, daß eine Einheit 91 zur Vorverarbeitung und Verdichtung für die Spektren des Gesamtspektrums 90 dient, aus dem in der Auswerteeinheit 92 z.B. die Kenngrößen PC 1 bis PC 10 berechnet werden. Bei der hierzu verwendeten Hauptkomponentenanalyse werden aus einer geeigneten Anzahl von Spektren, beispielsweise zwischen drei und zehn Spektren, sogenannte Scores, zwecks Datenreduktion gebildet. Daraus werden die Eingangsgrößen PC1,..., PCn insbesondere des Modells gemäß Figur 4 berechnet.

Die Kenngrößen PC 1 bis PC 10 fließen in das Zustandsmodell 93 und in das Prozeßmodell 94 ein, wobei zusätzlich diskrete mechanische und/oder chemische Eigenschaften und die Prozeßbeschreibung das Zustandsmodell zum Prozeßmodell ergänzt. Vom Zustandsmodell 93 werden die Produkteigenschaften der Kochflüssigkeit und aus dem Prozeßmodell 94 die Produkteigenschaften des Zellstoffes abgeleitet.

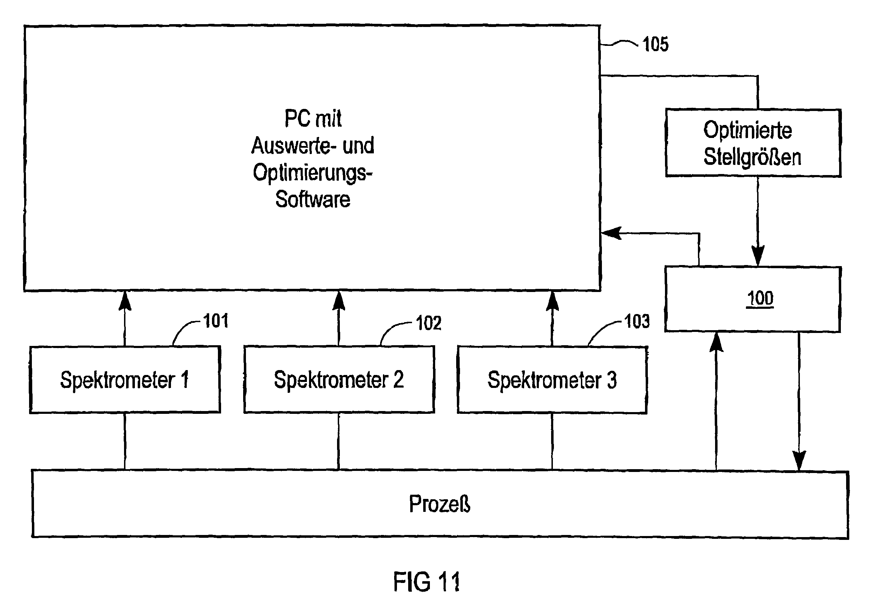

Figur 11 zeigt, wie mit entsprechender Auswerte- und Optimierungssoftware anhand eines Rechners 105 in ein vorhandenes Prozeßleitsystem eingegriffen werden kann. Dabei werden optimierte Stellgrößen erzeugt, die ein bekanntes Automatisierungsgerät 100 als Prozeßleitsystem beaufschlagen, das mit der eigentlichen Anlage zur Durchführung des Prozesses in Wechselwirkung steht. Im Prinzip werden also die üblichen Anlagen durch mehrere Spektrometer 101 bis 103 und ein zugehöriges Software-Paket, das auf dem Rechner 105 abläuft, ergänzt.

## Patentansprüche

1. Verfahren zur Prozeßführung und zur Prozeßoptimierung beim Herstellen von Zellstoff durch Kochen von Ausgangsstoffen mit Kochflüssigkeit in einem Zellstoffkocher unter Einsatz wenigstens eines Zustandsmodells und/oder Prozeßmodells, mit folgenden Merkmalen:
a) an mindestens einer Stelle werden an der Kochflüssigkeit und/oder am Zellstoff kontinuierliche Spektren von elektromagnetischer Strahlung und/oder kontinuierliche Spektren von mechanischen Eigenschaften gemessen,
b) durch mathematische Auswertung der kontinuierlichen Spektren werden Kenngrößen (PCl...PCn) für die Kochflüssigkeit und/oder den Zellstoff ermittelt,
c) aus den Kenngrößen (PCl...PCn) und Labormessungen der Produkteigenschaften wird das Zustandsmodell aufgestellt und/oder es wird zusätzlich mit Prozeßeigenschaften das Prozeßmodell aufgestellt,
d) mit Hilfe des so gebildeten Zustandsmodells, mit oder ohne Prozeßmodell, werden optimierte Stellgrößen für ein vorhandenes Prozeßleitsystem gebildet.

2. Verfahren nach Anspruch 1, , **dadurch gekennzeichnet, daß** zusätzlich mit Prozeßeigenschaften das Prozeßmodell aufgestellt wird und mit Hilfe des so gebildeten Prozeßmodells optimierte Stellgrößen für ein vorhandenes Prozeßleitsystem gebildet werden.

3. Verfahren nach Anspruch 1 oder 2, **dadurch gekennzeichnet, daß** an mindestens einer Stelle an der Kochflüssigkeit und/oder am Zellstoff diskrete physikalische und/oder chemische Eigenschaften erfaßt werden.

4. Verfahren nach Anspruch 3, **dadurch gekennzeichnet, daß** die diskreten physikalischen und/oder chemischen Eigenschaften zur Aufstellung des Zustandsmodells und ggfs. des Prozeßmodells verwendet werden.

5. Verfahren nach Anspruch 1 oder 2, **dadurch gekennzeichnet, daß** bei Wellenlängen der elektromagnetischen Strahlung zwischen 100 nm und 400 µm gemessen wird.

6. Verfahren nach Anspruch 5, **dadurch gekennzeichnet, daß** die elektromagnetische Strahlung als Absorptions-, Emissions-, Lumineszenz- oder als Raman-Spektrum erfaßt wird.

7. Verfahren nach Anspruch 5, **dadurch gekennzeichnet, daß** die elektromagnetische Strahlung in Transmission, direkter oder diffuser Reflexion oder gedämpfter Totalreflexion (ATR) erfaßt wird.

8. Verfahren nach Anspruch 1 oder 2, **dadurch gekennzeichnet, daß** als kontinuierliche Spektren der mechanischen Eigenschaften die Faserlängenverteilung oder die Siebfraktionen der Fasern herangezogen werden.

9. Verfahren nach Anspruch 3, **dadurch gekennzeichnet, daß** als diskrete physikalische und/oder chemische Eigenschaften die elektrische Leitfähigkeit, die Temperatur, die Durchflüsse, der Alkaligehalt und/oder die Sulfidität der Kochflüssigkeit erfaßt wird.

10. Verfahren nach Anspruch 1 oder 2, **dadurch gekennzeichnet, daß** an einer vorgegebenen Anzahl von Spektren eine Hauptkomponentenanalyse durchgeführt und zur Datenreduktion eine entsprechende Anzahl von Scores ausgewählt wird, und daß daraus die Kenngrößen für die Modellbildung ermittelt werden.

11. Verfahren nach Anspruch 10, **dadurch gekennzeichnet, daß** die Spektren vorverarbeitet und verdichtet werden, und daß die spezifischen Kennwerte der Spektren, insbesondere die Hauptkomponenten, zur Beschreibung des Produktzustandes ausgewählt werden und unmittelbar in das Zustandsmodell eingegeben werden.

12. Verfahren nach Anspruch 10, **dadurch gekennzeichnet, daß** die Spektren vorverarbeitet und verdichtet werden, daß die spezifische Kenngrößen der Spektren, insbesondere die Hauptkomponenten, in das Zustandsmodell eingebracht werden, und daß am Ausgang des Zustandsmodells die Produkteigenschaften gebildet und unmittelbar in das Prozeßmodell eingegeben werden.

13. Verfahren nach Anspruch 1 oder 2, **dadurch gekennzeichnet, daß** für die Modellbildung ungeeignete Spektren durch Plausibilitätsprüfung eliminiert werden.

14. Verfahren nach einem der vorhergehenden Ansprüche, **dadurch gekennzeichnet, daß** zur Modellbildung Neuronale Netze und/oder Fuzzy-Logik eingesetzt werden.

15. Verfahren nach Anspruch 1 oder 2, **dadurch gekennzeichnet, daß** ein kontinuierlicher Kocher zur Zellstoffherstellung eingesetzt wird.

16. Verfahren nach Anspruch 1 oder 2, **dadurch gekennzeichnet, daß** ein diskontinuierlicher Kocher zur Zellstoffkochung eingesetzt wird.

17. Verfahren nach Anspruch 15 oder Anspruch 16, **dadurch gekennzeichnet, daß** die durch Auswertung der Spektren erhaltenen Kenngrößen zur Steuerung und/oder Regelung des Kochers herangezogen werden.

18. Verfahren nach Anspruch 17, **dadurch gekennzeichnet, daß** zur Steuerung und/oder Regelung des Kochers die Qualitätsparameter des fertigen Zellstoffes, insbesondere die Reißlänge und/oder die Kappazahl und/oder die Viskosität, modelliert werden.

19. Verfahren nach Anspruch 18, **dadurch gekennzeichnet, daß** die Modellansätze neben der Vorhersage der Produktqualität auch zur Berechnung der Chemikalieneinsätze herangezogen werden.

20. Verfahren nach Anspruch 19, **dadurch gekennzeichnet, daß** Teilmodelle entsprechend den eingesetzten Holzarten und den Sollwerten der Qualitätsparameter gebildet werden.

21. Verfahren nach Anspruch 19, **dadurch gekennzeichnet, daß** der Modellansatz mit den Qualitätsparametern in einer Prozeßoptimierung eingesetzt wird.

22. Verfahren nach einem der vorhergehenden Ansprüche, **dadurch gekennzeichnet, daß** eine Kostenfunktion gebildet wird, die mit einem Optimierer durch geeignete Variation der Stellgrößen optimiert wird.

23. Verfahren nach Anspruch 20, **dadurch gekennzeichnet, daß** die Optimierung durch genetische Algorithmen erfolgt.

24. Verfahren nach Anspruch 21, **dadurch gekennzeichnet, daß** als Kostenfunktion eine Kostenfunktion für die Produktionskosten und/oder eine Gewinnfunktion eingesetzt wird.

25. Verfahren nach einem der vorhergehenden Ansprüche, **dadurch gekennzeichnet, daß** ein dynamisches Modell zur Überprüfung der durch ein statisches Modell optimierten Stellgrößen eingesetzt wird, wobei als dynamisches Modell ein neuronales Netz verwendet wird.

26. Verfahren nach einem der vorhergehenden Ansprüche, **dadurch gekennzeichnet, daß** das Modell und/oder die Teilmodelle online trainiert werden.

27. Verfahren nach einem der vorhergehenden Ansprüche, **dadurch gekennzeichnet, daß** durch rechnergestützte Auswahl aller informationstragenden Daten eine Überprüfung von erhaltenen Ergebnissen durchgeführt wird ("Novelty Detection").

28. Verfahren nach Anspruch 27, **dadurch gekennzeichnet, daß** bei Vorliegen nichtkonsistenter Ergebnisse ein Nachtrainieren erfolgt.

29. Vorrichtung zur Durchführung des Verfahrens nach einem der Ansprüche 1 bis 27, bestehend aus wenigstens einem Spektrometer (101, 102, 103) zum Messen von kontinuierlichen Spektren, aus einem Digitalrechner (105) zur mathematischen Auswertung der kontinuierlichen Spektren zwecks Ermittlung der Kenngrößen (PC1 ... PCn) und zur Aufstellung des Zustandsmodells und/oder des Prozeßmodells aus den Kenngrößen (PC1 ... PCn) und gegebenenfalls den diskreten physikalischen und/oder chemischen Eigenschaften als Prozeßeigenschaften, sowie aus einem Prozeßleitsystem (100) zur Prozeßoptimierung zum Herstellen von Zellstoff unter Verwendung von optimierten Stellgrößen.

## Claims

1. Method for process management and process optimization in the production of pulp by cooking starting materials with cooking liquid in a pulp digester using at least one state model and/or process model, having the following features:
a) continuous spectra of electromagnetic radiation and/or continuous spectra of mechanical properties are measured at at least one location on the cooking liquid and/or on the pulp,
b) characteristic quantities (PC1 ... PCn) are determined for the cooking liquid and/or the pulp through mathematical evaluation of the continuous spectra,
c) the state model is compiled from the characteristic quantities (PC1 ... PCn) and laboratory measurements and/or the process model is additionally compiled using process properties,
d) optimized correcting variables are formed for an existing process control system by means of the thus formed state model, with or without a process model.

2. Method according to Claim 1, **characterized in that** the process model is additionally compiled using process properties and optimized correcting variables are formed for an existing process control system by means of the thus formed process model.

3. Method according to either of Claims 1 or 2, **characterized in that** discrete physical and/or chemical properties are recorded at at least one location on the cooking liquid and/or on the pulp.

4. Method according to Claim 3, **characterized in that** the discrete physical and/or chemical properties are used to compile the state model and, if applicable, the process model.

5. Method according to either of Claims 1 or 2, **characterized in that**, in the case of electromagnetic radiation, wavelengths of between 100 nm and 400 µm are measured.

6. Method according to Claim 5, **characterized in that** the electromagnetic radiation is recorded as an absorption, emission, luminescence or Raman spectrum.

7. Method according to Claim 5, **characterized in that** the electromagnetic radiation is recorded in transmission, direct or diffuse reflection or attenuated total reflection (ATR).

8. Method according to either of Claims 1 or 2, **characterized in that** the fibre length distribution or the screen fractions of the fibres are used as continuous spectra of the mechanical properties.

9. Method according to Claim 3, **characterized in that** the electrical conductivity, the temperature, the flow rates, the alkali content and/or the sulphidity of the cooking liquid are recorded as discrete physical and/or chemical properties.

10. Method according to either of Claims 1 or 2, **characterized in that** a principal component analysis is performed on a predefined number of spectra and a corresponding number of scores is selected for the purpose of data reduction, and from these are determined the characteristic quantities for model formation.

11. Method according to Claim 10, **characterized in that** the spectra are preprocessed and compressed, the specific characteristic quantities of the spectra, particularly the principal components, are selected for the purpose of describing the product state and entered directly into the state model.

12. Method according to Claim 10, **characterized in that** the spectra are preprocessed and compressed, the specific characteristic quantities of the spectra, particularly the principal components, are entered in the state model, and the product properties are formed at the output of the state model and entered directly into the process model.

13. Method according to either of Claims 1 or 2, **characterized in that** spectra which are unsuitable for model formation are eliminated through plausibility checking.

14. Method according to any one of the preceding Claims, **characterized in that** neural networks and/or fuzzy logic is used for the purpose of model formation.

15. Method according to either of Claims 1 or 2, **characterized in that** a continuous-process digester is used for pulp production.

16. Method according to either of Claims 1 or 2, **characterized in that** a batch-process digester is used for pulp cooking.

17. Method according to either of Claims 15 or 16, **characterized in that** the characteristic quantities obtained through evaluation of the spectra are used for open-loop and/or closed-loop control of the digester.

18. Method according to Claim 17, **characterized in that** the quality parameters of the finished pulp, particularly the breaking length and/or the kappa number and/or the viscosity, are modelled for the purpose of open-loop and/or closed loop control of the digester.

19. Method according to Claim 18, **characterized in that**, in addition to being used to predict the product quality, the model formulations are also used to calculate the chemical applications.

20. Method according to Claim 19, **characterized in that** sub-models are formed according to the types of wood used and the setpoint values of the quality parameters.

21. Method according to Claim 19, **characterized in that** the model formulation is used with the quality parameters in a process optimization.

22. Method according to any one of the preceding Claims, **characterized in that** a cost function is formed which is optimized, by means of an optimizer, through appropriate variation of the correcting variables.

23. Method according to Claim 20, **characterized in that** the optimization is effected through genetic algorithms.

24. Method according to Claim 21, **characterized in that** there is used as a cost function a cost function for the production costs and/or a profit function.

25. Method according to any one of the preceding Claims, **characterized in that** a dynamic model is used to verify the correcting variables optimized by a static model, a neural network being used as a dynamic model.

26. Method according to any one of the preceding Claims, **characterized in that** the model and/or the sub-models are trained online.

27. Method according to any one of the preceding Claims, **characterized in that** a verification of obtained results ("novelty detection") is performed through computer-aided selection of all information-bearing data.

28. Method according to Claim 27, **characterized in that** retraining is performed if inconsistent results are present.

29. Facility for performing the method according to any one of Claims 1 to 27, consisting of at least one spectrometer (101, 102, 102) for measuring continuous spectra, a digital computer (105) for mathematically evaluating the continuous spectra for the purpose of determining the characteristic quantities (PC1 ... PCn) and for compiling the state model and/or the process model from the characteristic quantities (PC1 ... PCn) and, if applicable, from the discrete physical and/or chemical properties, as process properties, and of a process control system (100) for process optimization for the purpose of producing pulp using optimized correcting variables.

## Revendications

1. Procédé pour conduire un processus et pour l'optimiser lors de la fabrication de cellulose par cuisson de substances de départ avec un liquide de cuisson dans un cuiseur de pâte en utilisant au moins un modèle d'état et/ou un modèle de processus, ayant les caractéristiques suivantes :
a) des spectres continus du rayonnement électromagnétique et/ou des spectres continus de propriétés mécaniques sont mesurés en au moins un point sur le liquide de cuisson et/ou sur la cellulose,
b) en évaluant mathématiquement les spectres continus, on détermine des grandeurs (PC1...PCn) caractéristiques du liquide de cuisson et/ou de la cellulose,
c) à partir des grandeurs (PC1...PCn) caractéristiques et des mesures de laboratoire des propriétés du produit, on établit le modèle d'état et/ou on établit en plus avec les propriétés du processus le modèle de processus,
d) à l'aide du modèle d'état ainsi formé avec le modèle de processus ou sans le modèle de processus, on forme des grandeurs réglantes optimisées pour un système de conduite de processus existant.

2. Procédé suivant la revendication 1, **caractérisé en ce que** l'on établit en plus avec des propriétés de processus le modèle de processus et, à l'aide du modèle de processus ainsi formé, on forme des grandeurs réglantes optimisées pour un système de conduite de processus existant.

3. Procédé suivant la revendication 1 ou 2, **caractérisé en ce qu'**au moins en un endroit du liquide de cuisson et/ou de la cellulose, on relève des propriétés physiques et/ou chimiques discrètes.

4. Procédé suivant la revendication 3, **caractérisé en ce que** l'on utilise les propriétés physiques et/ou chimiques discrètes pour établir le modèle d'état et le cas échéant le modèle de processus.

5. Procédé suivant la revendication 1 ou 2, **caractérisé en ce que** l'on mesure le rayonnement électromagnétique à des longueurs d'onde comprises entre 100 nm et 400 µm.

6. Procédé suivant la revendication 5, **caractérisé en ce que** l'on relève le rayonnement électromagnétique sous la forme de spectres d'absorption, de spectres d'émission, de spectres de luminescence et de spectres Raman.

7. Procédé suivant la revendication 5, **caractérisé en ce que** l'on relève le rayonnement électromagnétique en transmission par réflexion directe ou par réflexion diffuse ou par réflexion totale amortie (ATR).

8. Procédé suivant la revendication 1 ou 2, **caractérisé en ce que** l'on utilise comme spectre continu des propriétés mécaniques, la répartition des longueurs de fibre ou les fractions granulométriques des fibres.

9. Procédé suivant la revendication 3, **caractérisé en ce que** l'on relève comme propriétés physiques et/ou chimiques discrètes la conductivité électrique, la température, les débits, la teneur en produits alcalins et/ou la teneur en sulfure du liquide de cuisson.

10. Procédé suivant la revendication 1 ou 2, **caractérisé en ce que** l'on effectue sur un nombre donné à l'avance de spectres une analyse des composantes principales et on choisit pour la réduction des données un nombre correspondant de scores et on en détermine les grandeurs caractéristiques pour la formation du modèle.

11. Procédé suivant la revendication 10, **caractérisé en ce que** l'on prétraite et compresse les spectres et **en ce que** l'on choisit les valeurs caractéristiques spécifiques des spectres, notamment les composantes principales pour décrire l'état du produit et on les introduit directement dans le modèle d'état.

12. Procédé suivant la revendication 10, **caractérisé en ce que** l'on prétraite et compresse les spectres **en ce que** l'on introduit des grandeurs caractéristiques spécifiques des spectres, notamment les composantes principales dans le modèle d'état, et **en ce que** l'on forme à la sortie du modèle d'état les propriétés du produit et on les introduit directement dans le modèle de processus.

13. Procédé suivant la revendication 1 ou 2, **caractérisé en ce que** pour la formation du modèle on élimine les spectres qui ne conviennent pas par un contrôle de plausibilité.

14. Procédé suivant l'une des revendications précédentes, **caractérisé en ce que** l'on utilise pour la formation du modèle des réseaux neuronaux et/ou une logique floue.

15. Procédé suivant la revendication 1 ou 2, **caractérisé en ce que** l'on utilise un cuiseur continu pour la préparation de la pâte.

16. Procédé suivant la revendication 1 ou 2, **caractérisé en ce que** l'on utilise un cuiseur discontinu pour faire cuire la pâte.

17. Procédé suivant la revendication 15 ou 16, **caractérisé en ce que** l'on tire parti des grandeurs caractéristiques obtenues en évaluant les spectres pour commander et/ou réguler le cuiseur.

18. Procédé suivant la revendication 17, **caractérisé en ce que** pour commander et/ou réguler le cuiseur, on modélise les paramètres de qualité de la cellulose finie, notamment la longueur de rupture et/ou l'indice kappa et/ou la viscosité.

19. Procédé suivant la revendication 18, **caractérisé en ce que** l'on tire parti des équations du modèle outre pour prédire la qualité du produit, également pour calculer les produits chimiques à engager.

20. Procédé suivant la revendication 19, **caractérisé en ce que** l'on forme des modèles partiels en fonction des types de bois utilisés et des valeurs de consigne des paramètres de qualité.

21. Procédé suivant la revendication 19, **caractérisé en ce que** l'on utilise l'équation du modèle ayant les paramètres de qualité dans une optimisation du processus.

22. Procédé suivant l'une des revendications précédentes, **caractérisé en ce que** l'on forme une fonction de coût que l'on optimise par un optimiseur par des variations appropriées des grandeurs réglantes.

23. Procédé suivant la revendication 20, **caractérisé en ce que** l'on effectue l'optimisation par des algorithmes génétiques.

24. Procédé suivant la revendication 21, **caractérisé en ce que** l'on utilise comme fonction de coût une fonction de coût pour les coûts de production et/ou une fonction de profil.

25. Procédé suivant l'une des revendications précédentes, **caractérisé en ce que** l'on utilise un modèle dynamique pour contrôler les grandeurs réglantes optimisées par un modèle statique en utilisant comme modèle dynamique un réseau neuronal.

26. Procédé suivant l'une des revendications précédentes, **caractérisé en ce que** l'on fait subir au modèle et/ou au modèle partiel un apprentissage en ligne.

27. Procédé suivant l'une des revendications précédentes, **caractérisé en ce que** l'on effectue par une sélection assistée par ordinateur de toutes les données portant de l'information un contrôle des résultats obtenus ("Novelty Detection").

28. Procédé suivant la revendication 27, **caractérisé en ce qu'**en présence de résultats non consistants, on effectue un nouvel apprentissage.

29. Dispositif pour la mise en oeuvre du procédé suivant l'une des revendications 1 à 27, constitué d'au moins un spectromètre (101, 102, 103) de mesure de spectre continu, d'un ordinateur (105) numérique d'évaluation mathématique des spectres continus en vue de déterminer des grandeurs (PC1...PCn) caractéristiques et d'établir le modèle d'état et/ou le modèle de processus à partir des grandeurs (PC1...PCn) caractéristiques et le cas échéant des propriétés physiques et/ou chimiques discrètes en tant que propriété du processus, ainsi que d'un système (100) de conduite de processus destiné à optimiser le processus pour produire de la cellulose en utilisant des grandeurs réglantes optimisées.
